# EUROPEAN PATENT APPLICATION

(11) **EP 2 383 096 A1**
(43) Date of publication of application: **02.11.2011**
(21) Application number: 11175803.3
(22) Date of filing: 07.03.2008
(51) Int. Cl.: B29C 41/08, B29C 41/20, B05B 13/02, A61F 2/82, B05B 1/28

(54) **Apparatus for making a vascular stent**

(30) Priority: 14.03.2007 US 685832
(62) Divisional of application: 08152466.2
(71) Applicant: Cordis Corporation, Miami Lakes, FL 33014 (US)
(72) Inventor: Cook, David, Erin, Ontario NOB 1TO (CA); Dave, Vipul Bhupendra, Hillsborough, NJ New Jersey 08844 (US); Johnstone, Stephen, Mississauga, Ontario L54 4A4 (CA); Leidner, Jacob, Toronto, Ontario M3H 1C8 (CA); White, Brian R, Etobicoke, Ontario M9W 3E2 (CA)
(74) Representative: Brown, George Laurence

(57) **Abstract**

The apparatus (100) for preparing a polymeric structure from which a number of medical devices may be constructed is described. The structures are preferably formed from bioabsorbable materials using low temperature fabrication processes, whereby drugs or other bio-active agents are incorporated into or onto the device and degradation of the drugs or other agents during processing is minimized.
The apparatus comprises a first platform (125) mounted to a surface; a first drive (124) mounted on the platform; a stage (102) having motion imparted thereto by the first drive; and a material dispenser (110) mounted in proximity to the stage such that material can be deposited onto the stage.

## Description

The invention generally relates to bioabsorbable drug delivery devices. In particular, a polymeric structure from which the device is constructed contains at least one agent dispersed evenly throughout the structure.

It is desirable to deliver therapeutic and biologically active agents to targeted areas within a vessel or duct. For example, devices such as stents are increasingly employed for delivering such agents during the repair of blood vessels narrowed or occluded by disease. Typically the stent is percutaneously routed to a treatment site and expanded to maintain or restore the patency of the conduit in which the stent is placed. Ideally, once placed within the vessel, passageway or duct, the stent will conform to the contours and functions of the blood vessel, passageway or duct in which the stent is deployed.

In order to prevent conditions such as restenosis wherein a vessel closes after placement of the stent therein, a stent can carry a therapeutic agent. Stents or other implantable devices may also serve as a means for delivering therapeutic agents to a localized area. Typically, therapeutic agents are coated onto a stent via a polymer having the therapeutic agent embedded in its matrix. Once in an aqueous environment the agent will elute from the polymer in a controlled manner. The polymer, however, will remain on the surface of the stent after the drug has been substantially eluted. This can cause adverse affects such as the formation of thrombosis. Moreover, it is not necessary for the stent to remain within the vessel or conduit after remodeling of the vessel.

Biodegradable materials may be employed to manufacture the stent, whereby the main body of the stent degrades in a predictably controlled manner. Stents of this type may further comprise therapeutic agents or other biologically active agents that are contained within the biodegradable materials from which the stent itself is constructed eliminating the need for a coating. This has the further advantage of allowing for more therapeutic agent to be delivered. The therapeutic agents are released as the biodegradable materials of the stent degrade.

US-5,464,450 (Buscemi) describes a biodegradable stent that is formed by mixing or solubilizing the drugs with the biodegradable polymer comprising the stent, dispersing the drug into the polymer during extrusion of the polymer, or by coating the drug onto an already formed film or fiber. A stent formed in this manner, however, is limited in the amount of therapeutic agent that may be included in the device. Another drawback to this approach is that therapeutic agent delivered from a stent or device formed in this manner tends to concentrate at a primary region of the device rather than delivering agent more uniformly along a length of the device. This limits the treatment of a targeted site where a diseased condition exists.

Typical methods of preparing biodegradable polymeric drug delivery devices, such as stents, include fiber spinning, film or tube extrusion or injection molding. All of these methods tend to use processing temperatures that are higher than the melting temperature of the polymers. For example, as described in Buscemi, melt-spinning a polymer into fibers requires temperatures from 60° ― 200° C. These temperatures are high relative to the temperatures at which most therapeutic agents are stable. There are few therapeutic agents, however, that are stable at these temperatures. Thus, the efficacy of the therapeutic agent will be compromised or the selection of therapeutic or biologically active agents limited.

In view of the above, a need exists for an apparatus and methods for forming a biodegradable device having increased amounts of therapeutic agent therein wherein the therapeutic agent is not degraded during formation of the structure.

An apparatus and method are provided for forming a bioabsorbable polymeric drug delivery device with increased and more effective drug delivery capacity that can also contain other additives or fillers such as radiopaque agents. The devices are formed from a structure, such as a tube, that is created from bioabsorbable polymers using low temperature fabrication processes. Preferred low temperature processes for preparing different structures such as films, foams, fibers and tubes include, for example, solution processing and extrusion, melt processing using solvents and plasticizers, processing from gels and viscous solutions, and super-critical fluid processing, whereby drugs that are not stable at high temperatures are able to be incorporated into the polymer forming the device.

Different processing methods can further include solvent extraction, coating, co-extrusion, wire-coating, lyophilization, spinning disk, wet and dry fiber spinning, electrostatic fiber spinning, and other processing methods known in the art. The preferred low temperature processes increases the amount or concentration of drugs or other agents that may be incorporated into the drug delivery devices made according to the systems and methods of the invention. Different geometries and performance characteristics of the drug delivery devices are achieved according to the different processes and materials used to make the devices.

In some embodiments, the drug delivery device is a stent comprised of bioabsorbable polymers with drugs or other bio-active agents and radiopaque markers incorporated therein. The stent is cut from a polymeric structure such as a tube. The materials used to construct the tube may comprise polymers, co-polymers, polymer blends and additives such as plasticizers and/or fillers. Therapeutic agents such as drugs or other bioactive materials are incorporated into the polymer matrix and/or coated onto the tube allowing any device formed from the tube to carry increased amounts of agent. Likewise, additives such as radiopaque markers may be provided in or on the tube. The combination of greater amounts of drugs, or other agents, for delivery from a device constructed from the tube with the radiopaque markers tends to improve the treatment of a targeted site, disease or condition and the visualization and placement of the device in the patient.

The bioabsorbable polymeric materials that comprise the stent or other device according to the systems and methods of the invention are chosen based on several factors, including degradation time, retention of the mechanical properties of the stent or other device during the active drug delivery phase of the device, and the ability of the bioabsorbable materials to be processed into different structures and via different methods. Other factors, including processing and material costs and availability, may also be considered.

In accordance with the invention a method for making a polymeric structure, such as a tube, comprises adding polymer(s) to a given solvent. The polymer and solvent mixture is tumbled with or without heat until the polymer dissolves completely in the solvent to provide a homogenous solution. Polymer formulations can be prepared using these solutions that may include radiopaque and/or therapeutic agents. These formulations may be further tumbled and mixed to prepare uniform dispersions. The polymer solution is then deposited onto a stage at room or higher temperature. The stage may be coated, for example with Teflon, to improve eventual removal there from.

The stage is dried after depositing the polymer solution onto it. If this drying phase is carried out in open air or in an atmosphere of inert gas, however, a non-uniform surface with drying lines may form on the tube. In order to eliminate these non-uniformities, drying may be carried out in a solvent-rich atmosphere. This reduces the rate of solvent evaporation and minimizes "skin" formation on the wet tube. This step can be carried out by keeping the coated stage rotating in a reservoir with the solvent or by blanketing the stage with inert gas rich in solvent vapor. After the first step of drying, the coated stage is transferred to a drying box where further drying takes place under a nitrogen atmosphere. Thereafter, drying is continued at an elevated temperature, e.g., 60-70°C in a nitrogen atmosphere. Drying under vacuum is also effective at this point. Solvent level is reduced and the structure is removed from the stage. Final removal of solvent can be carried out by different drying methods. Once the tube is dried it may be formed into a medical device, for example, laser cut into a stent.

An apparatus for forming the polymer structure comprises a stage or column that is driven at variable speeds. For example, if the polymeric structure being formed comprises a tube, the stage or column is rotated at variable speeds. At least one delivery system deposits material(s) onto the column at different flow rates. The delivery system includes a nozzle or other type of head that can focus the materials onto a selected point of the column. The delivery system and column are movable relative to each other. Varying the speed of movement of the column, the movement of the delivery system relative to the column and/or the rate at which the material is deposited onto the column will determine the thickness of the structure being formed.

The types of bioabsorbable polymers contemplated by the systems and methods of the invention include, but are not limited to, bulk or surface erosion polymers that are hydrophilic or hydrophobic, respectively, and combinations thereof. These polymers tend to help control the drug delivery aspects of the stent or other drug delivery device. Other bioabsorbable polymeric materials that may comprise the stent or other drug delivery device according to the systems and methods of the invention are shape memory polymers, polymer blends, and/or composites thereof that contribute to retaining the mechanical integrity of the device until drug delivery is completed.

The apparatus may be used in a method comprising the steps of:
preparing a solution of a polymeric material and at least one therapeutic agent using a solvent;
dispensing a first layer of the solution onto a stage contained within a chamber;
coating the length and surfaces of the stage;
providing a solvent within the chamber;
partially removing the solvent from the material by rotating the stage for a first period of time within the chamber;
removing the stage having partially dried material thereon from the chamber; and
placing the stage in a (drying) environment to further remove the solvent from the material.

The polymeric material preferably comprises a bioabsorbable polymer. The bioabsorbable polymer may comprise poly(α ― hydroxy esters). The bioabsorbable polymer may comprise poly(lactide-co-glycolide) copolymers. The bioabsorbable polymer may comprise a blend of at least two bioabsorbable polymers. The bioabsorbable polymer may be blended with at least one plasticizer.

The solution preferably further comprises at least one radiopaque agent.

The therapeutic agent preferably comprises an anti-proliferative.

The step of preparing the solution preferably comprises:
dissolving at least one polymeric material in a solvent to make a first solution;
dissolving at least one therapeutic agent in a solvent to make a second solution; and
mixing the first and second solutions;

The method may further comprise the step of dispensing at least one additional layer of the solution onto the first layer.

The method may further comprise the step of providing the solvent to the chamber by evaporating the solvent in an inert gas and supplying the evaporated solvent into the chamber.

The solvent may be located at the bottom of the chamber.

The above and other features of the invention, including various novel details of construction and combinations of parts, will now be more particularly described with reference to the accompanying drawings and claims. It will be understood that the various exemplary embodiments of the invention described herein are shown by way of illustration only and not as a limitation thereof. The principles and features of this invention may be employed in various alternative embodiments without departing from the scope of the invention.

Embodiments of the invention will now be described by way of example with reference to the accompanying drawings, in which:
Figure 1 is a perspective view of an apparatus for forming a structure in accordance with the present invention.
Figure 2 is a top view of the apparatus of Figure 1 for forming a structure in accordance with the present invention.
Figure 3 is a side view of an alternate embodiment of the apparatus of Figure 1.
Figure 4 is side view of yet another alternate embodiment of the apparatus of Figure 1.
Figure 5 is a perspective view of an alternate embodiment of an apparatus for forming a structure in accordance with the present invention.

A device is constructed from a structure comprised of bioabsorbable materials. The structure can include at least one agent, for example at least one drug or bioactive material and/or a radiopaque material or fillers. The structure, such as a tube, can be prepared by solution-based processes using solvents as by, for example, fiber spinning (dry and wet spinning), electrostatic fiber spinning, spinning disk (thin films with uniform thickness), lyophilization, extrusion and co-extrusion, co-mingled fibers, supercritical fluids, solvent cast films, or solvent cast tubes, wherein low temperature processing is preferred in order to minimize degradation of therapeutic agents or other bio-active agents that are incorporated into the matrix of bioabsorbable polymeric materials comprising the device. To this end, processing methods may comprise forming the device from bioabsorbable polymeric materials via low temperature, solution-based processes, for example using solvents.

The structure prepared according to the invention can contain agents such that any device constructed there from will be disease specific and designed for local or regional therapy, or a combination thereof. The therapeutic or bioactive agents are ideally controllably released, wherein the rate of release depends on either or both of the degradation rate of the bioabsorbable polymers comprising the device and the nature of the drugs or other agents. The rate of release can thus vary from minutes to years as desired. Surface erosion polymers or bulk erosion polymers, for example, can be used as the bioabsorbable polymer in order to better control the drug delivery there from.

Surface erosion polymers are typically hydrophobic with water labile linkages. Hydrolysis tends to occur quickly on the surface of such polymers with no water penetration in bulk. The drug release rate from devices comprised of such surface erosion polymers can thus be varied linearly while maintaining the mechanical integrity of the device. The initial strength of such surface erosion polymers tends to be low however, and often such surface erosion polymers are not readily available commercially. Nevertheless, examples of surface erosion polymers that could be used to help vary the drug delivery rate of a device according to the systems and methods of the invention include polyanhydrides such as poly (carboxyphenoxy hexane-sebacic acid), poly (fumaric acid-sebacic acid), poly (carboxyphenoxy hexane-sebacic acid), poly (imide-sebacic acid)(50-50), poly (imide-carboxyphenoxy hexane)(33-67), and polyorthoesters (diketene acetal based polymers).

Bulk erosion polymers, on the other hand, are typically hydrophilic with water labile linkages. Hydrolysis of bulk erosion polymers tends to occur at more uniform rates across the polymer matrix of the device. As a result, bulk erosion polymers release initial bursts of drugs during breakdown of the polymer matrix during absorption. Bulk erosion polymers exhibit superior initial strength and are readily available commercially.

Examples of bulk erosion polymers usable with the drug delivery devices according to the system and methods of the invention include poly (α-hydroxy esters) such as poly (lactic acid), poly (glycolic acid), poly (caprolactone), poly (p-dioxanone), poly (trimethylene carbonate), poly (oxaesters), poly (oxaamides), and their co-polymers and blends. Some commercially readily available bulk erosion polymers and their commonly associated medical applications include poly (dioxanone) [PDS® suture available from Ethicon, Inc., Somerville, NJ], poly (glycolide) [Dexon® sutures available from United States Surgical Corporation, North Haven, CT], poly (lactide)-PLLA [bone repair], poly (lactide/glycolide) [ Vicryl® (10/90) and Panacryl® (95/5) sutures available from Ethicon, Inc., Somerville, NJ], poly (glycolide/caprolactone (75/25) [ Monocryl® sutures available from Ethicon, Inc., Somerville, NJ], and poly (glycolide/trimethylene carbonate) [ Maxon® sutures available from United States Surgical Corporation, North Haven, CT].

Other bulk erosion polymers are also usable with the drug delivery devices according to the systems and methods of the invention, for example, tyrosine derived poly amino acid [examples: poly (DTH carbonates), poly (arylates), and poly (imino-carbonates)], phosphorous containing polymers [examples: poly (phosphoesters) and poly (phosphazenes)], poly (ethylene glycol) [PEG] based block co-polymers [PEG-PLA, PEG-poly (propylene glycol), PEG-poly (butylene terphthalate)], poly (α -malic acid), poly (ester amide), and polyalkanoates [examples: poly (hydroxybutyrate (HB) and poly (hydroxyvalerate) (HV) co-polymers].

The structures may be formed from combinations of surface and bulk erosion polymers in order to achieve desired physical properties and to control the degradation mechanism and drug release there from as a function of time. For example, two or more polymers may be blended in order to achieve desired physical properties, device degradation rate and drug release rate. Alternatively, the structure is formed from a bulk erosion polymer that is coated with a drug containing a surface erosion polymer. For example, the drug coating can be sufficiently thick that high drug loads can be achieved, and the bulk erosion polymer may be made sufficiently thick that the mechanical properties of the device are maintained even after all of the drug has been delivered and the surface eroded.

While the degradation and drug release factors are considered in choosing the bioabsorbable polymers that are to comprise the structure according to the systems and methods of the invention, maintaining the mechanical integrity and resilience of the device is also a factor to consider. In this regard, shape memory polymers that are bioabsorbable help a device to maintain, or remember, its original shape after deployment of the device in the patient. Shape memory polymers are characterized as phase segregated linear block co-polymers having a hard segment and a soft segment. The hard segment is typically crystalline with a defined melting point, and the soft segment is typically amorphous with a defined glass transition temperature. The transition temperature of the soft segment is substantially less than the transition temperature of the hard segment in shape memory polymers. A shape in the shape memory polymer is memorized in the hard and soft segments of the shape memory polymer by heating and cooling techniques in view of the respective transition temperatures as the artisan should appreciate.

The selection of the bioabsorbable polymeric material used to comprise the drug delivery device according to the invention is determined according to many factors including, for example, the desired absorption times and physical properties of the bioabsorbable materials, and the geometry of the drug delivery device.

In order to provide materials having high ductility and toughness, such as is often required for orthopedic implants, sutures, stents, grafts and other medical applications including drug delivery devices, the bioabsorbable polymeric materials may be modified to form composites or blends thereof. Such composites or blends may be achieved by changing either the chemical structure of the polymer backbone, or by creating composite structures by blending them with different polymers, fillers and plasticizers. Plasticizers such as low molecular weight poly(ethylene glycol) and poly(caprolactone), and citrate esters can be used. Any additional materials used to modify the underlying bioabsorbable polymer should preferably be compatible with the main polymer system. The additional materials also tend to depress the glass transition temperature of the bioabsorbable polymer, which renders the underlying polymer more ductile and less stiff.

As an example of producing a composite or blended material for the drug delivery device, blending a very stiff polymer such as poly (lactic acid), poly (glycolide) and poly (lactide-co-glycolide) copolymers with a soft and ductile polymer such as poly (caprolactone) and poly(dioxanone) tends to produce a material with high ductility and high stiffness. An elastomeric co-polymer can also be synthesized from a stiff polymer and a soft polymer in different ratios. For example, poly(glycolide) or poly(lactide) can be copolymerized with poly(caprolactone) or poly(dioxanone) to prepare poly(glycolide-co-caprolactone) or poly(glycolide-co-dioxanone) and poly(lactide-co-caprolactone) or poly(lactide-co-dioxanone) copolymers. These elastomeric copolymers can then be blended with stiff materials such as poly(lactide), poly(glycolide) and poly(lactide-co-glycolide) copolymers to produce a material with high ductility. Alternatively, terpolymers can also be prepared from different monomers to achieve desired properties. Macromers and other cross-linkable polymer systems can be used to achieve the desired properties. Such properties are conducive to a drug delivery stent device according to systems and methods of the invention. Of course, the underlying polymer could also be blended with a stiffer polymer to produce a material having stiffer properties, as might be useful in the case of an orthopedic implant having growth factors or other bio-active agents or drugs delivered there from according to the systems and methods of the invention.

The therapeutic agents, such as drugs or other bio-active agents, delivered by the drug delivery devices constructed from the structure may include rapamycin, statins and taxol, or any of the other drugs or bio-active agents not otherwise identified herein. The drugs or other agents may reduce different indications such as restenosis, vulnerable plaque, angina and ischemic stroke, for example, particularly where the device is a stent. Growth factors, such as fibro-blasts and vascular endothelial growth factors can also be used in lieu of, or together with, the drugs. Such growth factors may be used for angiogenesis, for example.

In addition to the various drugs identified above, the drugs or other agents incorporated into the device can also include cytostatic and cytotoxic agents, such as, heparin, sirolimus, everolimus, tacrolimus, biolimus, paclitaxel, statins and cladribine. The various drugs or agents can be hydrophobic or hydrophilic as appropriate. In some of the examples set forth below, sirolimus was the drug incorporated into the drug delivery devices.

Other drugs or other bio-active agents usable with the drug delivery devices made according to the systems and methods described herein include:
antiproliferative/antimitotic agents including natural products such as vinca alkaloids (i.e., vinblastine, vincristine, and vinorelbine), paclitaxel, epidipodophyllotoxins (i.e., etoposide, teniposide), antibiotics (dactinomycin (actinomycinD) daunorubicin, doxorubicin and idarubicin), anthracyclines, mitoxantrone, bleomycins, plicamycin (mithramycin) and mitomycin, enzymes (L-asparaginase which systemically metabolizes L-asparagine and deprives cells which do not have the capacity to synthesize their own asparagines); antiplatelet agents such as G(GP) 11_{b}/111ₐ inhibitors and vitronectin receptor antagonists; antiproliferative/antimitotic alkylating agents such as nitrogen mustards (mechlorethamine, cyclophosphamide and anolgs, melphalan, chlorambucil), ethylenimines and methylmelamines (hexamethylmelamine and thiotepa), alkyl sulfonaates-busulfan, nirtosoureas (carmustine (BCNU) and analogs, streptozocin), trazenes-dacarbazinine (DTIC); antiproliferative/antimitotic antimetabolites such as folic acid analogs (methotrexate), pyrimidine analogs (flourouracil, floxuridine, and cytarabine), purine analogs and related inhibitors (mercaptopurine, thioguanine, pentostatin and 2-chlorodeoxyadenosine {cladribine}); platinum coordination complexes (cisplatin, carboplatin), procarbazine, hydroxyurea, mitotane, aminglutethimide; hormones (i.e., estrogen); anticoagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory; antisecretory (breveldin); anti-inflammatory: such as adrenocortical steroids (cortisol, cortisone, fludrocortisone, prednisone, prednisolone, 6 α-methylprednisolone, triamcinolone, betamethasone, and dexamethasone), non-steroidal agents (salicylic acid derivatives i.e., aspirin; para-aminphenol derivatives i.e., acetominophen; indole and indene acetic acids (indomethacin, sulindac, and etodalac), heteroaryl acetic acids (tolmetin, diclofenac, and ketorolac), arylpropionic acids (tometin, diclofenac, and ketorolac), arylpropionic acids (ibuprofen and derivatives), anthranilic acids (mefenamic acid, and meclofenamic acid), enolic acids (piroxicam, tenoxicam, phenylbutazone, and oxyphenthatrazone), nabumetone, gold compounds (auranofin, aurothioglucose, gold sodium thiomalate); immunosuppressives: (cyclosporine, tacrolimus (FK-506), sirolimus (rapamycin), azathioprine, mycophenolate (mofetil); angiogenic agents: vascular endothelial growth factor (VEGF), fibroblast growth factor (FGF); angiotensin receptor blockers; nitric oxide donors; anti-sense oligionucleotides and combinations thereof; cell cycle inhibitors, mTOR inhibitors, and growth factor receptor signal transduction kinase inhibitors; retenoids; cyclin/CDK inhibitors; HMG co-enzyme reductase inhibitors (statins); and protease inhibitors.

The amount of drugs or other agents incorporated within the structure can range from 0 to 99% (% weight of the device). The drugs or other agents can be incorporated into the device in different ways. For example, the drugs or other agents can be coated onto the device after the device has been formed, wherein the coating is comprised of bioabsorbable polymers into which the drugs or other agents are incorporated. Alternatively, the drugs or other agents can be incorporated into the matrix of bioabsorbable materials comprising the device. The drugs or agents incorporated into the matrix of bioabsorbable polymers can be in an amount the same as, or different than, the amount of drugs or agents provided in the coating techniques if desired. These various techniques of incorporating drugs or other agents into, or onto, the drug delivery device may also be combined to optimize performance of the device, and to help control the release of the drugs or other agents from the device.

Where the drug or agent is incorporated into the matrix of bioabsorbable polymers comprising a device constructed from the structure of the present invention, for example, the drug or agent will release by diffusion and during degradation of the device. The amount of drug or agent released by diffusion will tend to release for a longer period of time than occurs using coating techniques, and can often more effectively treat local and diffuse lesions or conditions. For regional drug or agent delivery such diffusion release of the drugs or agents is effective as well.

Preferred low temperature processes of forming the drug delivery devices according to the systems and methods of the invention include solution processing and supercritical fluid processing techniques. These processes include solvent extraction, coating, wire-coating, extrusion, co-extrusion, fiber-spinning including electrostatic fiber-spinning, lyophilization and other techniques that incorporate drugs or other bio-active agents that are unstable at high temperatures into the matrix of bioabsorbable polymeric materials that will comprise the drug delivery device. For drugs or agents that are stable at high temperature, different melt processing techniques may instead be used to incorporate the drugs or agents into the matrix of bioabsorbable polymers that comprise the device. Alternatively, the drugs or agents may be sprayed, dipped, or coated onto the device after formation thereof from the bioabsorbable polymers. In either case, the polymer matrix, and drug or agent blend when provided, is then converted into a structure such as fibers, films, discs/rings or tubes, for example, that is thereafter further manipulated into various geometries or configurations as desired.

Different processes can alter the morpohological characteristics of the bioabsorbable polymer being used to form the structure of the present invention. For example, when dilute solutions of polymers are stirred rapidly, the polymers tend to exhibit polymer chains that are generally parallel to the overall axis of the structure. On the other hand, when a polymer is sheared and quenched to a thermally stable condition, the polymer chains tend to elongate parallel to the shear direction. Still other morphological changes tend to occur according to other processing techniques. Such changes may include, for example, spherulite to fibril transformation, polymorphic crystal formation change, re-orientation of already formed crystalline lamellae, formation of oriented crystallites, orientation of amorphous polymer chains and/or combinations thereof.

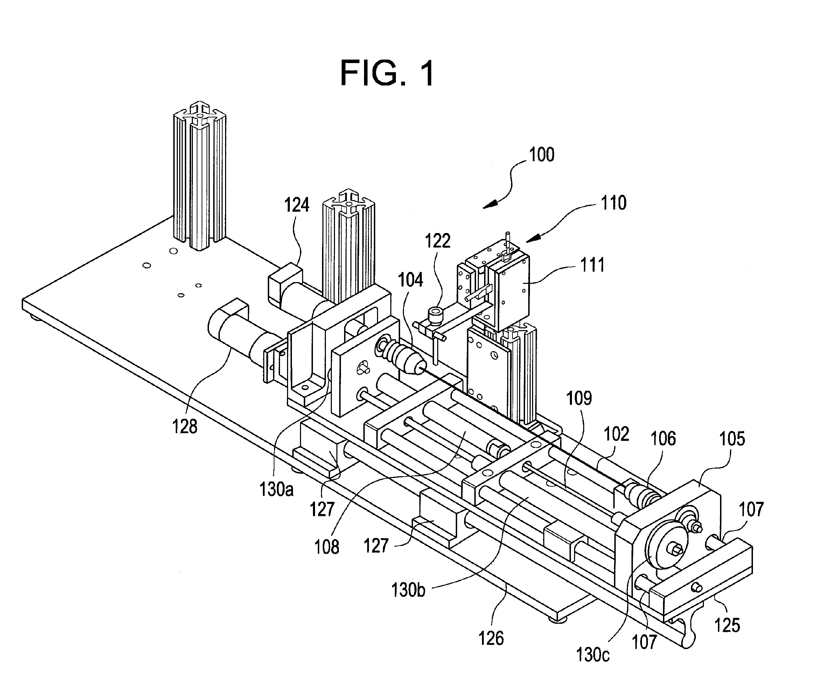

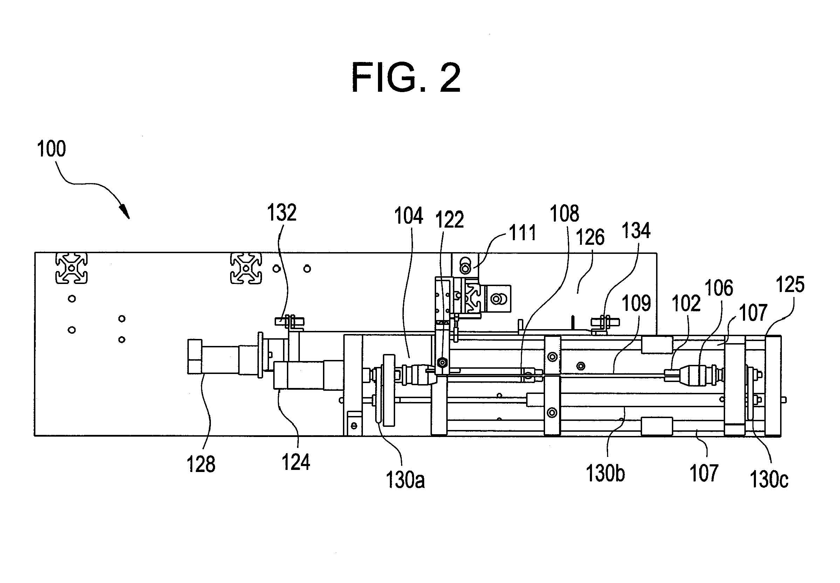

An apparatus that can form a structure such as a tube will be described with reference to Figures 1-2. The apparatus 100 comprises a first platform 125 mounted on slides 127 to a second platform 126. A platform drive 128 moves the first platform 125 relative to the second platform 126. A stage 102 is located in proximity to a dispense tip 122 which is in fluid communication with a material supply apparatus supported on mount 111 which is mounted onto the second platform 126. Material is deposited onto the stage 102 from the dispense tip 122, as will be described in greater detail below. A drive 124 imparts motion to the stage 102 so that the polymeric or other material will be deposited at a desired location or thickness there along. Drive 124 may comprise a motor that imparts rotational motion to the stage 102. In addition, the drive 124 may include a piston or push device that imparts longitudinal motion and a tensioning force to the stage 102.

The stage 102 is mounted to the first platform 125 by placing the proximal and distal ends of the stage 102 into a first grip 104 and second grip 106 respectively. The grips 104 and 106 are moveable in a direction dictated by drive 124. For example, if drive 124 imparts rotational movement, grip 104 is connected to the shaft of the drive 124 and grip 106 is mounted to support 105. Rotational motion is imparted to grip 106 through transmission elements 130a-c. The drive 124 rotates element 130a, which in turn rotates element 130b. Element 130b rotates element 130c, which in turn drives the grip 106. The transmission 130 ensures that if stage 102 has a small thickness or diameter it will not twist while being rotated due to differing rotational speeds of the first 104 and second 106 grips. If drive 124 includes a piston or other push device, grips 104 and 106 may be mounted onto the first stage 125 to slide longitudinally thereon. For example, support 105 is mounted on rails 107 for longitudinal movement there along. In one embodiment of the invention each element 130a-c include and/or comprise gears whereby motion is imparted from one element to the other. In an alternative embodiment of the invention, elements 130a-c include and/or comprise a pulley whereby motion is imparted from one element to the next by belts that link the pulleys. In yet another alternative embodiment a combination of gears, belts and pulleys are used to impart motion from one element 130a-c to the next.

Stage 102 may experience deformation as material is deposited thereon. Maintaining the stage 102 under tension will alleviate, if not eliminate, such deformations. A tensioning device 108 is mounted on the first platform 125 and includes a push rod 109 having its distal end connected to the support 105. As the stage 102 experiences deformation, rod 109 will push the support 105 and, consequently the grip 106, distally from grip 104 along rails 107. This will place the stage 102 under increased tension and eliminate any sagging or other deformations. The tensioning device 108 may comprise a pneumatic cylinder, a solenoid or, alternatively a belt assembly connected to a drive motor.

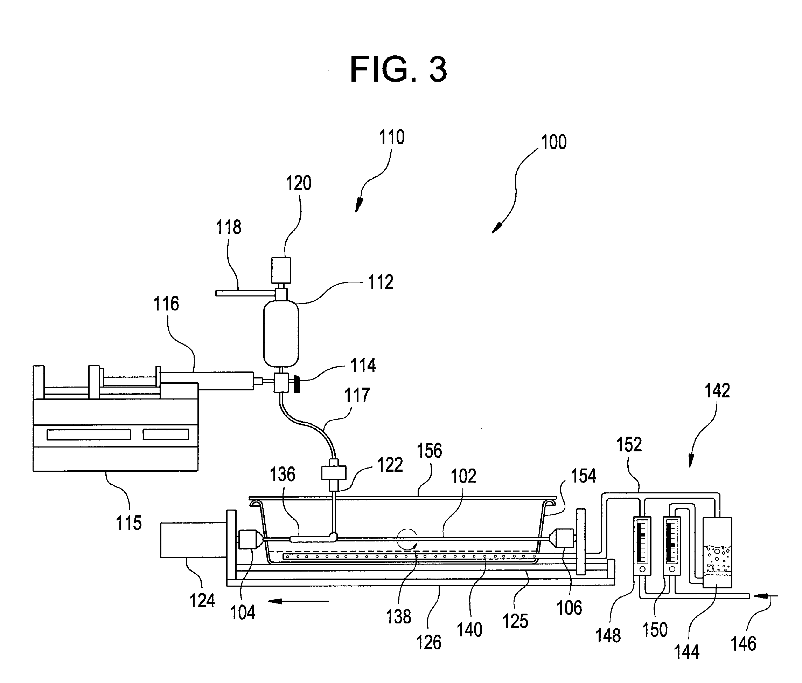

As shown in Figure 3, a dispensing apparatus 110 is positioned above the stage 102. Preferably, as shown in Figures 1-2, the dispensing apparatus 110 is supported above stage 102 by mount 111. The dispensing apparatus comprises a syringe 116 driven by pump 115 and a reservoir 112 for supplying material to the dispense tip 122. A valve 114 directs the flow of material from the syringe 116 to the dispense line 117. During refilling of the syringe 116, the valve 114 is switched to guide material from the reservoir 112 into the syringe 116. In particular, the pump 115 is switched to refill and nitrogen pressurization through line 118 into the reservoir pushes material into the syringe 116, refilling it. After refilling, the valve 114 is switched back to guide material through the dispense line 117. The casting solution in reservoir 112 may also be mixed with a stirrer powered by a stirrer motor 120 to ensure homogeneity in the casting solution or to redisperse solids that might result from settling.

In order to cast or create a structure, the pressure to the syringe 116 is stabilized and the drive 124 for the stage 102 is activated imparting rotational motion thereto. The dispensing tip 122 is extended forward towards stage 102. The proximal grip 104 is directly driven from the drive 124 while the distal grip 106 is driven through transmission 130, as described above. After the dispense tip 122 is fully extended, the platform drive 128 is turned on. The pump 115 is actuated such that the syringe 116 supplies material to the dispense tip 122 via the dispense line 117. Material 136 flows onto the stage 102, which rotates and moves longitudinally to evenly distribute material thereon. The platform drive 128 moves the first platform 125 in a direction towards drive 128 while the dispense tip 122 remains stationary. A sensor 132 is positioned on the second platform 126. When the proximal end of the first platform 125 reaches sensor 132, the drive 128 stops. Although the stage 102 is tensioned prior to depositing material thereto, it may be necessary to apply additional tension to the stage 102. Another sensor may be included which monitors the strain of the stage 102 as material 136 is deposited thereon. If the strain is greater than anticipated, then tensioning device 108 may be further actuated to move support 105 distally.

After material has been deposited onto the stage 102, the dispensing tip 122 is retracted and the stage 102 continues to rotate until the material 136 deposited onto the stage 102 has dried enough to allow for the stage to be removed from grips 104 and 106 without any spillage. In order to remove the stage 102, the drive 124 is stopped, tension is relieved from the stage 102 by retracting tensioning device 108, the grips 104 and 106 are loosened and the stage 102 can be removed. The platform is then returned to the home position by reversing the direction of the platform drive 128 and stopping it when the distal end of the platform reaches sensor 134.

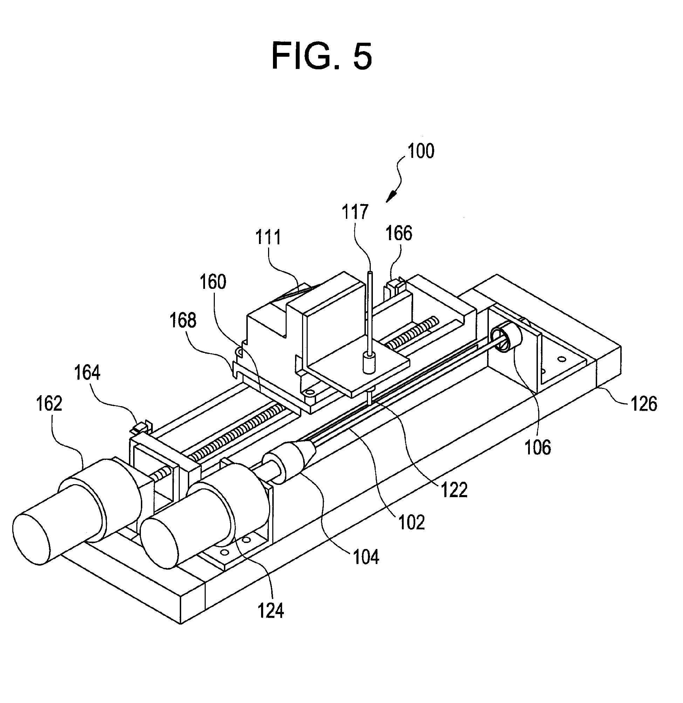

Figure 5 shows an alternate embodiment 200 of the apparatus, wherein a third platform 260 is mounted onto the second platform 226 so as to slide relative to the second platform 226. A support 261 is mounted atop second platform 226 and has third platform 260 mounted thereto so as to slide there along. A drive 262 imparts motion to the third platform 260 via drive screw 272 to move the third platform 260 along support 261. The dispense support 211 is mounted to the third platform 260 and the dispense tip 222 is mounted on support 211 so as to be in proximity to the stage 202. The stage 202 is rotated and the drive 262 actuated to move third platform 260 as material flows from the dispense tip 222 and is deposited onto stage 202. Movement of the platform 260 is halted when a sensor stop located on the leading edge of the third stage, not shown in the drawings, is detected by sensor 266. After the conclusion of depositing material on the stage 202, the drive 262 is actuated to return the platform 260 to the home position, which is reached when sensor 264 detects sensor stop 268.

The embodiment shown in Figure 5 and in Figures 1-2 may be combined to provide for longitudinal movement of both the stage 102 and dispense device 110 by, for example, mounting the third platform 260 on first platform 125 in the same manner as the first platform 125 is mounted on the surface by rails 107. Alternatively, both the first 125 and third platform 260 may be mounted on rails located on the second platform 126. Any of these embodiments allows the amount of material 136 deposited on the stage 102 to be precisely controlled by altering the rate of movement of the platform(s) 125 and/or 260 in combination with the rotational speed of the stage 102, or by altering the flow rate of the dispensing device 110, or any combination thereof.

After the stage 102 has been removed it is placed in a chamber to dry the material 136 deposited thereon. This takes place in several steps. The first step is partial drying immediately after depositing the material 136 onto the stage ― if this drying phase is carried out in open air or in an atmosphere of inert gas, a non-uniform surface with drying lines forms on the dried material. In order to eliminate these, drying has to be carried out in a solvent-rich atmosphere. This reduces the rate of solvent evaporation and minimizes "skin" formation on the wet tube. This step can be carried out by keeping the coated stage rotating in a reservoir with the solvent or by blanketing the stage with inert gas rich in solvent vapor. After the first step of drying, the coated stage is transferred to a drying box where further drying takes place under a nitrogen atmosphere. The next step is carried out at an elevated temperature, e.g., 60-70°C in a nitrogen atmosphere in an oven. Drying under vacuum is also effective at this step. At the end of this step the solvent level is about 7% by weight and the material forms a structure that is removed from the stage 102. Final removal of solvent from the structure is carried out using different drying methods including carbon dioxide extraction and high temperature oven drying.

One example of this process uses a dioxane solvent resident in the bottom of a chamber to a depth of approximately 1 cm. The coated stage 102 is rotated in the solvent chamber for at least 45 minutes. During this period, relatively little air flows over the solvent chamber so as to minimize the drying rate. The coated stage 102 is removed from the chamber and placed in a nitrogen purge chamber for room temperature drying for at least 1.5 hours. The purge rate is fairly low (0.5 to 1 SCFH). The stage is then placed into oven at 40° ― 70° C for about 10 hours. The dried material 136 is now a solid structure that can be removed from the stage, for example, by twisting sections of the structure allowing it to be slid off of the stage 102. Thereafter, the structure may be formed into a medical device. For example, if the stage 102 was a tube, the structure will be a tube that may be laser cut to form an implantable stent.

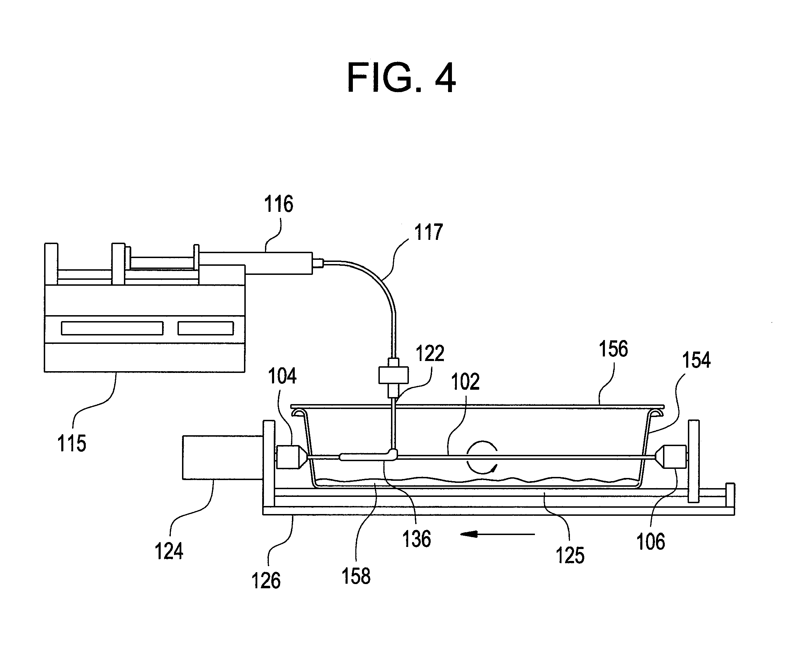

As shown in Figures 3-4, the apparatus can be modified to combine the step of depositing material onto stage 102 with the some, or all, of the drying process. For example, as shown in Figure 4, the stage 102 is mounted such that it is located within a casting chamber 154. The chamber 154 has a cover 156 that is slotted allowing for passage of the dispense tip 122 there through and movement of the dispense tip 122 along the length of stage 102. The bottom of the chamber 154 has a solvent to a depth of about 0.75 cm. The presence of a solvent controls the rate at which the material 136 dries. For example, if the material dries too quickly it would result in an uneven surface in the final structure.

Yet another embodiment is shown in Figure 3 where the stage 102 is placed in chamber 154 and an inert gas supplied thereto. An inert gas sparging apparatus 142 is used to maintain an appropriate solvent level in the atmosphere surrounding the stage 102 to regulate the rate of the drying process. The pressurized inert gas flows from a supply into the apparatus 142 along line 146. The gas is split into two flows controlled by rotameters 148 and 150. The flow from rotameter 150 is directed to a sparging bottle 144 filled with solvent. The inert gas becomes saturated with solvent at this point. The inert gas flows are then re-combined in feed line 152 before entering the casting chamber 154. By adjusting the ratio of rotameter flows it is possible to adjust the solvent content in the combined flows between zero and full saturation. The combined flow enters the chamber 154 and is dispersed through a perforated tube 140 running the length of the solvent chamber 154. A perforated plate 138 enhances the dispersion of the saturated inert gas ensuring that the solvent content is the same throughout the chamber. With this arrangement it is possible to maintain better control of the drying process and any oxygen and moisture, which can be detrimental to the product, are excluded from the structure forming process.

The principles of the present invention may be further illustrated by the following example wherein the material used to prepare the solution for forming the structure and the method of forming the structure is described.

### I. Tubes Prepared from PLA/PGA 85/15 with Radiopaque and Therapeutic Agent.

### (a) Typical Method for the Preparation of Solution

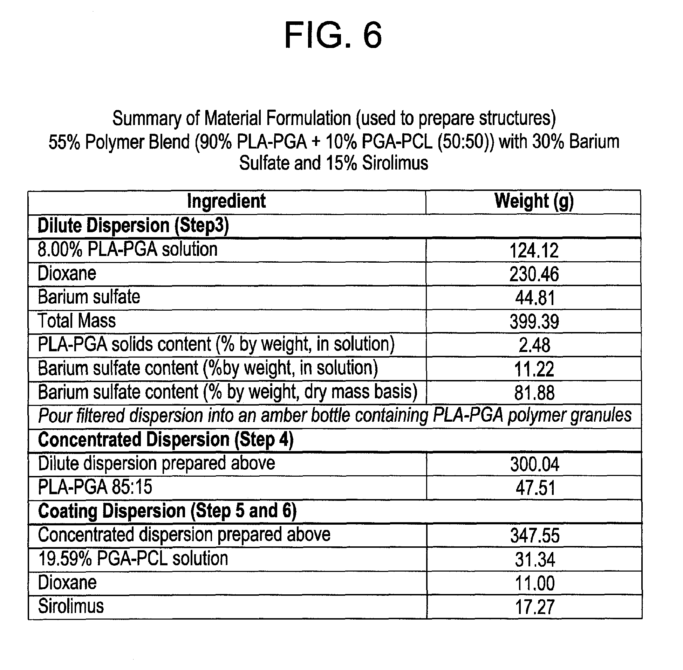

The high solid dispersion summarized in Figures 6-7 was prepared using the general procedure described below:
1. A PLA-PGA solution (polymer content 8.0% by weight) was prepared. PLA-PGA granules were placed into a pre-cleaned amber bottle. Thereafter, dioxane was added to the bottle wherein the target total polymer solids content is 8.0% w/w in dioxane. The headspace in the bottle was filled with nitrogen gas and the bottle sealed. Finally, the bottle is tumbled at 55 ± 3°C (rotational speed about 5 rev/min).
2. A PGA-PCL solution was prepared (polymer content 19.59% by weight). In particular, PGA-PCL granules were placed into a pre-cleaned amber bottle and dioxane added to the bottle. The target total polymer solids content was 19.59% w/w in dioxane. Nitrogen was added to the headspace in the bottle and the bottle sealed. Finally, the bottle was tumbled at 55 ± 3°C (rotational speed about 5 rev/min).
3. A dilute PLA-PGA/barium sulfate-containing dispersion is prepared wherein a mixer equipped with the larger mixing head was used to disperse the barium sulfate. Dioxane was placed into a pre-cleaned clear wide mouth jar. The 8% PLA-PGA solution, prepared in Step 1, was placed into the same jar. The jar was swirled to spread polymer solution. Barium sulfate, a radiopaque marker, was then placed into the same jar. The mixing head was positioned just above the base of the jar. Nitrogen is flowed into the jar at a rate of 5 psig in a blanket-dispersion. The solution is mixed at 4000-8000 rpm for about 7 minutes keeping the nitrogen blanket over the dispersion throughout mixing. The dispersion was filtered through a 25µm pore size stainless steel mesh with wiper attachment under a slight positive nitrogen pressure (2-5 psig).
4. Concentrated PLA-PGA solution was prepared by addition of the filtered dilute dispersion of Step 3 to PLA-PGA polymer granules. PLA-PGA granules were placed directly into an amber bottle pre-cleaned with dioxane. The filtered dilute dispersion prepared in Step 3 is added to the same amber bottle. The headspace in the bottle is purged with nitrogen and then the bottle is sealed. The bottle is tumbled at 55 ± 3°C (rotational speed about 5 rev/min).
5. The PGA-PCL solution of Step 2 was added to the concentrated PLA-PGA dispersion of Step 4. The headspace of the bottle containing the mixture of the two solutions was filled with nitrogen and sealed. The bottle was tumbled at 55 ± 3°C (rotational speed about 5 rev/min).
6. A final dispersion was prepared with therapeutic agent being added to the dispersion of Step 5. In particular, a desired amount of therapeutic agent, for example sirolimus, was placed into a pre-cleaned glass beaker. The agent was transferred directly into the bottle containing the concentrated dispersion of Step 5. Dioxane was added to the bottle and the bottle was shaken to disperse the agent throughout the dispersion of Step 5. The headspace of the bottle was filled with nitrogen gas and the bottle sealed. The bottle was tumbled at room temperature (rotational speed about 7 rev/min). Weights and compositional information are summarized in the Figure 6 below. The barium sulfate content of the dilute dispersion was determined by ashing.

### (b) Deposition of Dispersion.

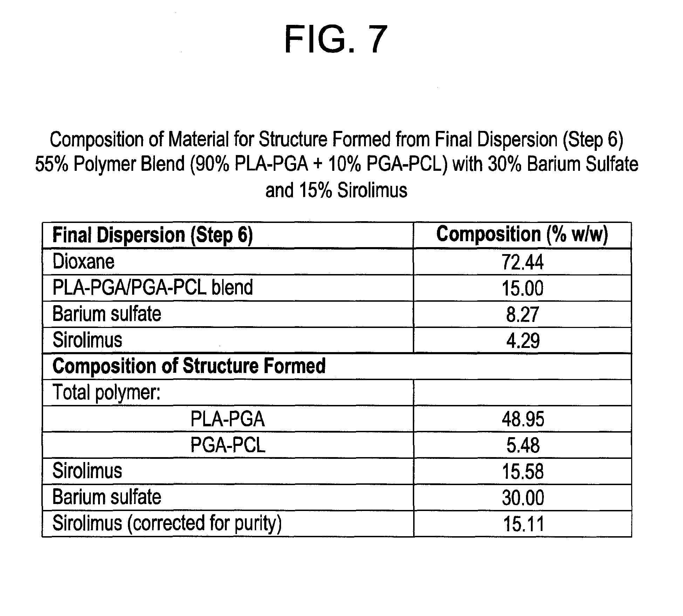

The structures were prepared from the final dispersion described in Figure 7 using the following process:
1. A stage made from 304-grade stainless steel with a PTFE coating was used. The stage finished OD was 0.0409" (1.04 mm) with a length of 14". The stage was rinsed with dioxane prior to being used.
2. A syringe was loaded with the dispersion and placed in an upright position to allow for air bubbles to rise to the top of the solution. If necessary, about 0.5 mL of coating solution was released to remove air bubbles.
3. The flow rate (mL/hr) required to meet the desired structure fabrication specifications was determined. For example, this was determined by performing three runs near the calculated flow rate, based on the casting platform diameter and solution solids content. The flow rate versus the outer diameter of the tube was plotted to finalize flow rate.
4. Dispersion from Step 6 was dispensed onto and along a rotating PTFE-coated stage contained in a dioxane-rich environment that reduced the drying rate. A cover was placed over the container once the stage was coated. The stage was rotated for 30 to 60 minutes as the coating partially dried in the main drying chamber.
5. The stage was removed from the chamber and stored in a box flushed with nitrogen for at least 30 minutes before drying at elevated temperature. The stage was maintained in a substantially upright position during drying at room temperature.

### (c) Drying the Stage to form a Structure

1. Drying began in the solvent-rich atmosphere of Step 4 of deposition of material onto the stage. This reduced the rate of solvent evaporation and minimizes "skin" formation on the wet tube. The coated stage was kept rotating in a reservoir with the solvent. Alternatively, the stage could have been blanketed with inert gas rich in solvent vapor.
2. After the first step of drying, the coated stage was transferred to a drying box where further drying took place under a nitrogen atmosphere.
3. The coated stage was dried at an elevated temperature, e.g., 60-70°C in a nitrogen atmosphere in an oven that may be under vacuum pressure. The solvent level in the structure , comprising the dried material on the stage, is about 7% by weight and structures are removed from the forming stages.
4. The material dried on the stage assumed a tubular form (the shape of the stage in this example). The tubular form is removed from the stage by placing one end of the stage in a vise and the tube (on the stage) slowly delaminated by compressing one end of the tube. Once delaminated, the tube was pulled off the stage and stored in an inert environment.
5. Final removal of solvent was carried out on the tubes by different drying methods including carbon dioxide extraction and high temperature oven drying.

### (d) Dimensional Measurement of Tubes

A Mitutoyo laser scan micrometer was used for measuring the outer diameter of tubes formed in the steps outlined above. Wall thickness was determined by the difference between the diameter of the stage and the outer diameter of the tube, which were measured using the laser scan micrometer. Typical process parameters and tube properties are as follows:

| | | |
|---|---|---|
| Stage rotational speed: | | 30-50 rpm |
| Dispense nozzle speed: | | 30-50 mm/min |
| Solution deposition rate: | | 8-12 mL/hr |
| Drying time: | | |
| Step 1 | 30 minutes | |
| Step 2 | Minimum of 2 hours under nitrogen | |
| Step 3 | 60°C for 6-12 hours | |
| Step 4 | (depending on extraction conditions) | |
| Tube dimensions: | | 1.45 ± 0.1 mm OD |

Although the present invention has been described above with respect to particular preferred embodiments, it will be apparent to those skilled in the art that numerous modifications and variations can be made to these designs without departing from the scope of the invention, which is defined by the appended claims.

## Claims

1. An apparatus comprising:
a first platform mounted to a surface;
a first drive mounted on the platform;
a stage having motion imparted thereto by the first drive; and
a material dispenser mounted in proximity to the stage such that material can be deposited onto the stage.

2. The apparatus of claim 1 wherein the first platform is movably mounted to the surface and preferably the first platform further comprises rails that are mounted in slides located on the surface

3. The apparatus of claim 2, wherein the first platform further comprises rails that are mounted in slides located on the surface and the apparatus further comprises a second drive for imparting motion to the first platform; and preferably wherein the surface comprises a second platform.

4. The apparatus of claim 1 wherein the first drive comprises a motor that imparts rotational motion to the stage and preferably further comprises a piston that imparts longitudinal motion to the stage.

5. The apparatus of claim 1 wherein the material dispenser is mounted so as to move relative to the first platform.

6. The apparatus of claim 1 wherein the material dispenser comprises:
a syringe,
a pump for actuating the syringe;
a dispense tip in fluid communication with the syringe and located in proximity to the stage so as to deposit material thereon.

7. The apparatus of claim 6 wherein the material dispenser further comprises a material storage reservoir and a stirrer located so as to mix the material stored within the reservoir, and the apparatus preferably comprises a valve which regulates the flow of material from the syringe to the dispense tip and from the reservoir to the syringe so as to maintain a constant supply of material to the dispense tip.

8. The apparatus of claim 1 wherein a first end of the stage is connected to the first drive by a first grip and second end of the stage is connected to a support by a second grip and preferably the second grip is rotated by the first drive.

9. The apparatus of claim 8 wherein the second grip is rotated by the first drive and the second grip is linked to the first drive through a transmission so as to ensure that the first and second grip rotate at the same speed.

10. The apparatus of claim 8 wherein the second grip is rotated by the first drive and the support is movably mounted to the first platform and preferably the apparatus further comprises a tensioning device that moves the support in a longitudinal direction along the first platform and the tensioning device optionally comprises an arm connected to the support driven by a piston located proximally to the arm.

11. The apparatus of claim 1 further comprising a chamber wherein the stage is at least partially located therein, and preferably wherein the material dispenser comprises a dispense tip in communication with a material source, the chamber having a cover that allows the dispense tip to be in proximity to the stage for dispensing material thereon.

12. The apparatus of claim 11, wherein the chamber contains a solvent for controlling the drying of material deposited onto the stage.

13. The apparatus of claim 11 wherein the material dispenser comprises a dispense tip in communication with a material source, the chamber having a cover that allows the dispense tip to be in proximity to the stage for dispensing material thereon and the chamber is supplied with an inert gas, preferably the inert gas supplied to the chamber is mixed with a solvent in a gas sparging device comprising a solvent source and at least two flow regulation devices that control the level of solvent in the inert gas, optionally the flow of inert gas in the chamber is dispersed through a perforated tube and a perforated plate located over the perforated tube.

14. The apparatus of any one of the preceding claims, wherein the stage is coated with Teflon.

15. The apparatus of any one of the preceding claims, wherein the stage is a column.
